(19) Europäisches Patentamt
European Patent Office
Office européen des brevets

(11) **EP 2 450 704 B1**

(12) **EUROPÄISCHE PATENTSCHRIFT**

(45) Veröffentlichungstag und Bekanntmachung des Hinweises auf die Patenterteilung:
**20.02.2013 Patentblatt 2013/08**

(51) Int Cl.:
***G01N 33/22*** *(2006.01)* ***G01F 1/00*** *(2006.01)*

(21) Anmeldenummer: **11008500.8**

(22) Anmeldetag: **21.10.2011**

(54) **Verfahren zur Bestimmung des Brennwertes von Brenngas in Gasnetzen, insbesondere in Regional- oder Verteilnetzen**

Method for determining the calorific value of fuel in gas networks, in particular in regional or distribution networks

Procédé de détermination de la valeur de combustion de gaz de combustion dans des réseaux de gaz, notamment dans des réseaux régionaux ou de distribution

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.11.2010 DE 102010050327**

(43) Veröffentlichungstag der Anmeldung:
**09.05.2012 Patentblatt 2012/19**

(73) Patentinhaber: **E.ON New Build & Technology GmbH**
**45896 Gelsenkirchen (DE)**

(72) Erfinder:
• **Schley, Peter**
**45133 Essen (DE)**
• **Schenk, Joachim**
**81369 München (DE)**

(74) Vertreter: **Harlacher, Mechthild**
**E.ON Ruhrgas AG**
**Brüsseler Platz 1**
**45131 Essen (DE)**

(56) Entgegenhaltungen:
**US-A1- 2006 212 249**

• **HERR ECKART ET AL: "Computer-Aided Allocation of Calorific Value Measurements Taken at the Feed Points of a Long-Distance Gas Grid to the Gas Volumes Withdrawn at Transfer Stations (Rechnergestutzte Zuordnung von an den Einspeisepunkten eines Ferngasnetzes vorgenommenen Brennwertmessungen zu den an Obergabestationen en", GAS- UND WASSERFACH, GAS - ERDGAS, MÜNCHEN : OLDENBOURG-INDUSTRIEVERL., 2001-, DE, Bd. 124, Nr. 3, 1. März 1983 (1983-03-01), Seiten 157-164, XP008147551, ISSN: 0016-4909**
• **"Praxisinformation_P2007/13 Gastransport/ Betriebswirtschaft", , 1. Januar 2007 (2007-01-01), XP55016825, Gefunden im Internet: URL:http://www.eichsfeldwerke.de/_data/Pra xisinformation_P2007_13.pdf [gefunden am 2012-01-18]**
• **"Gasbeschaffenheitsrekonstruktion Produktinformation apptech Reko", , 17. Juli 2009 (2009-07-17), XP55015842, Gefunden im Internet: URL:http: //www.appliedtechnologies.de/PDF Beschreibung/Produktbeschreibung apptech REKO.pdf [gefunden am 2012-01-06]**
• **PETER ULBIG ET AL: "DETERMINATION OF THE CALORIFIC VALUE OF NATURAL GAS BY DIFFERENT METHODS", THERMOCHIMICA ACTA, ELSEVIER SCIENCE PUBLISHERS, AMSTERDAM, NL, Bd. 382, 1. Januar 2002 (2002-01-01), Seiten 27-35, XP002639490, ISSN: 0040-6031**

EP 2 450 704 B1

- BECK HANS PETER ET AL: "Nachbildung nicht gemessener Abnahmen eines Gasverteilnetzes mit Hilfe eines Messgroessenbeobachters", GAS- UND WASSERFACH, GAS - ERDGAS, MÜNCHEN : OLDENBOURG-INDUSTRIEVERL., 2001-, DE , Bd. 148 1. Januar 2007 (2007-01-01), Seiten 270-280, XP008147098, ISSN: 0016-4909 Gefunden im Internet: URL:-

**Beschreibung**

Die Erfindung betrifft ein Verfahren zur Bestimmung des Brennwertes von Brenngas, insbesondere von Erdgas, in Gasnetzen, insbesondere in Regional- oder Verteilnetzen mit mindestens zwei Einspeisestellen, einer Mehrzahl von Netzknoten und mehreren Ausspeisestellen.

Gasnetze für die öffentliche Gasversorgung werden in Femleitungsnetze, Regional- und Verteilnetze bzw. Ortsnetze unterschieden. In Femleitungsnetzen beträgt der Druck des Gases typischerweise 60 bar, in Regionalnetze ca.16 bar und in Verteil- bzw. Ortsnetzen ca. 1 bar. Netzknoten sind Punkte im Gasnetz, an denen sich Gasströme verzweigen und/oder vermischen.

Erdgas kann je nach Lagerstätte einen unterschiedlichen Brennwert aufweisen. Erdgas mit verhältnismäßig niedrigen Brennwerten wird als L-Gas bezeichnet. Erdgas mit höheren Brennwerten als H-Gas.

Die Bestimmung des Brennwertes in Regional- oder Verteilnetzen beruht in der Regel auf Messwerten, die der Vorlieferant für die Einspeisestellen zur Verfügung stellt. Das DVGW-Arbeitsblatt G685 DVGW Technische Regel - Gasabrechnung. April 1993 legt fest, dass der zur Abrechnung verwendete Brennwert des Kunden nicht mehr als 2% von dem tatsächlich anstehenden Brennwert abweichen darf. Bei Mehrfacheinspeisung mit Erdgasen unterschiedlicher Qualität gilt diese Anforderung als erfüllt, wenn die mengengewichtete Jahresmittelwerte der eingespeisten Brennwerte nicht mehr als 2% voneinander abweichen.

Bislang wurde seitens der Gasversorgungsunternehmen auf die Einhaltung dieser Randbedingungen hinsichtlich der Einspeisung geachtet. Durch die Liberalisierung auf dem Energiemarkt, die zunehmend Mehrfacheinspeisung sowie starke Brennwertschwankungen zur Folge hat, wird diese Praxis erschwert. Wird bei Mehrfacheinspeisung die vorgenannte Anforderung nicht erfüllt, so muss der Gasnetzbetreiber für den entsprechenden Abrechnungszeitraum den niedrigsten vorgekommenen Brennwert - d. h. den für den Kunden günstigsten Fall - ansetzen ("Best"-Abrechnung).

Ferner wird in zunehmendem Maße aufbereitetes Biogas in Gasnetze für Erdgas eingespeist. Im Sinne der erwähnten 2%-Regel wird derzeit das eingespeiste Biogas entsprechend konditioniert und bei Einbindung in H-Gas-Netze durch eine Zumischung von Propan aus fossilen Quellen im Brennwert angepasst. Diese Lösung treibt Investitions- und Betriebskosten in die Höhe und widerspricht dem Ziel der Biogasnutzung als regenerative Energie.

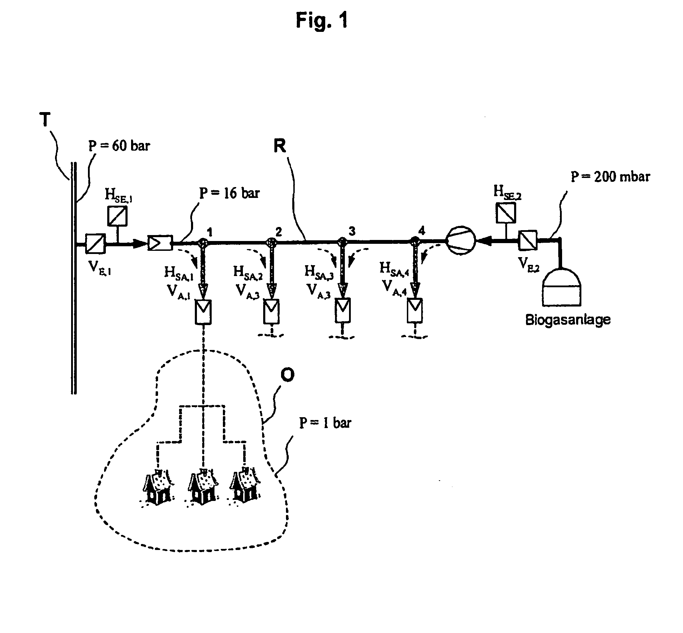

Die Thematik wird durch ein in Fig. 1 dargestelltes Beispiel verdeutlicht. Fig. 1 zeigt:

ein Transportnetz (T), das typischerweise bei einem Druck von 60 bar betrieben wird

ein Regional- oder Verteilnetz (R), das typischerweise bei einem Druck von 16 bar betrieben wird und

ein Ortsnetz (O), das typischerweise bei einem Druck von 1 bar betrieben wird.

In das Regionalnetz wird von zwei Seiten eingespeist. Zum einen wird von dem Transportnetz (T) ein Erdgas mit dem Brennwert $H_{SE,1}$, zum anderen wird ein Biogas mit dem Brennwert $H_{SE,2}$ eingespeist. An vier Ausspeisestellen wird dann das Gas von dem Regionalnetz an die Ortsnetze übergeben. Da die Ortsnetze nicht untereinander verbunden sind, kann man den Brennwert der Ausspeisestelle auch für das nachgeschaltete Ortsnetz zugrunde le gen. In dem dargestellten Beispiel werden die Ausspeisestellen 1 und 2 ausschließlich von dem Erdgas mit dem Brennwert $H_{SE,1}$ versorgt — d. h. $H_{SA,1} = H_{SA,2} = H_{SE,1}$. Die Ausspeisestelle 4 wird ausschließlich von dem Biogas versorgt; d. h. $H_{SA,4} = H_{SE,2}$. Das Gas an der Ausspeisestelle 3 ergibt sich aus einer Mischung der beiden Einspeiungen, d. h. hier kann nicht direkt einer der beiden Einspeisebrennwerte zugeordnet werden. In Abhängigkeit der eingespeisten Mengen kann sich der Bereich dieser Mischzone verschieben; man spricht in diesem Fall von einer Pendelzone.

Das dargestellte Beispiel verdeutlicht, dass bei Einspeisungen von mehreren Gasen unterschiedlicher Brennwerte, den Ausspeisestellen nicht in jedem Fall jeweils die richtigen Einspeisebrennwerte zugeordnet werden können. Eine korrekte Abrechnung kann folglich nicht gewährleistet werden. Alternativ müsste der Brennwert an allen Ausspeisestellen direkt gemessen werden, was mit erheblichen Kosten für die Installation und das Betreiben einer Messgeräteinfrastruktur verbunden wäre. Dabei ist zu berücksichtigen, dass reale Gasnetze in der Regel deutlich komplexer sind und wesentlich mehr Ausspeisestellen haben, als in dem Beispiel in Fig. 1 dargestellt wurde.

Für Fernleitungsnetze bzw. Transportnetze haben sogenannte "Brennwert-Rekonstruktionssysteme", mit denen der Brennwert zu Abrechnungszwecken an allen Ausspeisestellen des Netzes auf Basis einer "Simulationsrechnung" berechnet wird, in den letzten Jahren zunehmend an Bedeutung gewonnen und sind Stand der Technik; PTB-Anforderung 7.64: Messgeräte für Gas Brennwertmessgeräte - Ermittlung von Abrechnungsbrennwerten und weiteren Gasbeschaffenheitsdaten mittels Zustandsrekonstruktion. PTB, Dezember 1999. und PTB-Prüfregeln, Band 28: Messgeräte für Gas, Brennwertmessgeräte Ermittlung von Abrechnungsbrennwerten und weiteren Gasbeschaffenheitsdaten mittels Zustandsrekonstruktion. PTB, Januar 1998.

Der Einsatz von Brennwert-Rekonstruktionssystem ist ferner beschrieben in Herr, Eckart et al: Rechnergestützte Zuordnung von an den Einspeisepunkten eines Ferngasnetzes vorgenommenen Brennwertmessungen zu den an Übergabestationen entnommenen Gasmengen. Gwf-gas/erdgas 124 (1983), S. 157-164 sowie in Altfeld, K.; Bödeker, J.; Frieling, H.; Schley, P.; Uhrig, M.: Modelling of Gas Flow in Pipelines for Tracking Gas Quality. Proceedings of the International Gas Research Conference, Paris, 2008.

In der Regel verfügen Regional- und Verteilnetz, im Gegensatz zu Fernleitungsnetzen, nicht über eine vollständige Messinfrastruktur. Insbesondere werden die Abnahmemengen an den Ausspeisestellen zum Ortsnetz (s. Fig. 1) in der Regel nicht gemessen. Deswegen konnte bisher für Regional- oder Verteilnetze keine rechnerische Verfolgung des Brennwerts mit einer ausreichend hohen Genauigkeit realisiert werden, so dass ein solches Verfahren von den zuständigen Behörden für Abrechnungszwecke anerkannt worden wäre.

Statistische Verfahren zur Abschätzung von Verbrauchsmengen - genauer gesagt der verbrauchten Energiemengen - auf Basis von Lastprofilverläufen sind aus der Literatur bekannt und werden eingesetzt um den zu erwartenden Gasabsatz bestimmter Verbrauchergruppen zu prognostizieren. Auch können solche Verfahren eingesetzt werden, um bei Endkunden dem zeitlichen Versatz zwischen dem Abrechnungszeitraum und dem Zeitraum der Zählerstandsablesung Rechnung zu tragen. Dies kann insbesondere dann von Bedeutung sein, wenn der Kunde den Gasversorger gewechselt hat und der genaue Zählerstand zum Zeitpunkt des Wechsels nicht vorliegt.

Im Auftrag von BGW (Bundesverband der deutschen Gas- und Wasserwirtschaft) und VKU (Verband kommunaler Unternehmen e. V.) hat die TU München temperaturgeführte Standardlastprofile für die Gasversorgung entwickelt ( Hellweg, M.: Entwicklung und Anwendung parametrisierter Standard-Lastprofile. Dissertation TU München, 2003). In zusammengefasster Form wurden die Ergebnisse dieser Dissertation vom BGW in "Praxisinformation P 2007/13 Gastransport/Betriebswirtschaft, 1. Januar 2007, veröffentlicht.

Über die Sigmoid-Funktion wird der relative Verbrauch h($\vartheta$) in Abhängigkeit der gewichteten Tagesmitteltemperatur dargestellt

$$h(\vartheta) = \frac{A}{1 + \left(\dfrac{B}{\vartheta - 40°C}\right)^{C}} + D \qquad (1)$$

Die Parameter A, B, C und D wurden für verschieden Lastprofiltypen (z. B. Einfamilienhaus, Mehrfamilienhaus, Gebietskörperschaften, Einzelhandel) festgelegt. Ferner lässt sich bei Kenntnis eines zurückliegenden Ableseintervalls - dem sogenannten Periodenverbrauch Q - für einen Kunden ein sogenannter Kundenwert KW (in kWh) ermitteln, der den spezifischen, auf einer mittleren Bezugstemperatur normierten Verbrauch des Kunden wiedergibt. Bei Kenntnis der prognostizierten Temperatur, die z. B. vom Wetteramt bereitgestellt werden kann, lässt sich schließlich der zu erwartende stündliche Energieverbrauch (in kWh) eines Kunden oder als Summenwert einer Kundengruppe wie folgt bestimmen

$$Q_h = KW \cdot h(\vartheta) \cdot F(d) \cdot SF(h, \vartheta) \qquad (2)$$

darin ist F(d) der Wochentagsfaktor und SF(h, $\vartheta$) der Stundenfaktor.

Auf Basis solcher statistischer Verfahren kann die Verbrauchsmenge nur grob abgeschätzt werden.

Vor dem oben beschriebenen Hintergrund besteht die Aufgabe der vorliegenden Erfindung darin, ein Verfahren bereitzustellen, mit dem der Brennwert von Brenngas an Ausspeisestellen von Gasnetzen mit mindestens zwei Einspeisestellen einfach und genau bestimmt werden kann, ohne dass hierfür ein großer Aufwand für zusätzliche Messgeräte betrieben werden muss.

Gelöst wird diese Aufgabe durch ein Verfahren der eingangs genannten Art, dadurch gekennzeichnet, dass

a) die Brennwerte ($H_{S,E}$) und Mengen ($V_E$) an den Einspeisestellen des Gasnetzes gemessen werden,
b) die Mengen ($V_A$) an den Ausspeisestellen des Gasnetzes auf Basis von Lastprofilen aus der Energiemenge $Q_h$ und dem Brennwert $H_{s,A}$ gemäß der Gleichung

$$V_{A,i} = \frac{Q_{h,i}}{H_{sA,i}} \qquad (6)$$

geschätzt und gemäß der Gleichung

$$V_A = \sum_i V_{A,i} \qquad (5)$$

summiert werden, wobei sich die Energiemenge $Q_h$ aus der Gleichung

$$Q_h = KW \cdot h(\vartheta) \cdot F(d) \cdot SF(h,\vartheta) \qquad (2)$$

ergibt und wobei KW der Kundenwert (in kWh), $h(\vartheta)$ der relative Verbrauch gemäß der Sigmoid-Funktion, F(d) der Wochentagsfaktor und SF(h, 9) der Stundenfaktor ist,
c) die in den Verfahrensschritten a) und b) ermittelten Werte zusammen mit topologischen Daten des Gasnetzes einer Auswerteeinheit zugeführt und die Brennwerte ($H_{S,A}$) an mindestens einer Ausspeisestelle rechnerisch bestimmt werden.

Mit dem erfindungsgemäßen Verfahren kann der Brennwert an Ausspeisestellen von Regional- oder Verteilnetzen sehr genau bestimmt werden. Die ermittelten Werte sind so genau, dass sie für Gasabrechnung für Endkunden verwendet werden können.

Die Formeln (2) und (6) können durch geeignete andere Formeln ersetzt werden, die zu gleichen Ergebnissen führen.

Im Folgenden wird die Bestimmung der Abnahmemengen von einzelnen Ausspeisestellen bzw. Netzknoten beschrieben:

Allgemein lässt sich für ein Leitungsnetz bzw. für einen Netzabschnitt folgende Volumenbilanz aufstellen:

$$V_E = V_A + \Delta V_{Netz} \qquad (3)$$

mit

$$V_E = \sum_i V_{E,i} \qquad (4)$$

und

$$V_A = \sum_i V_{A,i} \qquad (5)$$

Mit Volumen ist hier das Gasvolumen im Normzustand ($t_n$ = 0°C, $p_n$ = 1,01325 bar) gemeint, im Folgenden auch als Normvolumen oder vereinfacht auch als Menge bezeichnet. Die gesamte ins Netz eingespeiste Menge $V_E$ ergibt sich aus der Summe aller in Netz eingespeisten Mengen $V_{E,i}$. Es wird davon ausgegangen, dass alle eingespeisten Mengen direkt gemessen werden.

Die gesamte aus dem Netz ausgespeiste Menge $V_A$ ergibt sich aus der Summe aller ausgespeisten Mengen $V_{A,i}$. An den Ausspeisestellen werden die Mengen in der Regel nicht direkt gemessen. Hier ergibt sich die Gesamtmenge an einer Ausspeisestelle aus der Summe aller Mengen der Endverbraucher, die über diesen Netzknoten versorgt werden. Um die Mengen der Endverbraucher zu ermitteln, können - wie zuvor beschrieben - die vorgenannten Lastprofile der TU-München herangezogen werden. Der nach Gleichung (2) ermittelte stündliche Energieverbrauch lässt sich mit Hilfe des volumenbezogenen Brennwertes $H_s$ in ein Normvolumen umrechnen.

$$V_{A,i} = \frac{Q_{h,i}}{H_{sA,i}} \qquad (6)$$

Da die genauen Brennwerte an den Ausspeisestellen zunächst noch nicht bekannt sind, wird im ersten Schritt für das gesamte Netz ein mittlerer Brennwert angenommen. Dieser kann z. B. als gewichteter Mittelwert aus den Einspeisebrennwerten und den Ausspeisemengen bestimmt werden.

Des Weiteren muss für die Aufstellung der Bilanz gemäß Gleichung (3) noch die Netzatmung - d. h. die Änderung des gesamten im Netz befindlichen Gasvolumens - berücksichtigt werden. Näherungsweise lässt sich das Gasvolumen im Netz unter Normbedingungen wie folgt bestimmen

$$V_{Netz} = \frac{p}{p_n} \cdot \frac{T_n}{T} \cdot V_{Geo} \qquad (7)$$

Die Änderung des Gasvolumens $\Delta V_{Netz}$ lässt sich bei Kenntnis des mittleren Netzdruckes zu Beginn $p(t_1)$ und zum Ende $p(t_n)$ des betrachteten Zeitraums (z. B. innerhalb einer Stunde) ermitteln. Der mittlere Netzdruck kann durch Messungen des Druckes an repräsentativen Stellen im Netz bestimmt werden. Die Temperatur kann dabei als konstant (z. B. 8 °C) angenommen werden.

$$\Delta V_{Netz} = V_{Netz}(t_i) - V_{Netz}(t_{(i-1)}) = (p_i - p_{(i-1)}) \cdot \frac{T_n}{p_n} \cdot \frac{V_{Geo}}{T} \qquad (8)$$

Eine bevorzugte Weiterbildung ist dadurch gekennzeichnet, dass

a) der mittlere Netzdruck ($p_m$) durch Messung des Druckes an repräsentativen Stellen in dem Gasnetz ermittelt wird und die zeitliche Änderung der im Gasnetz befindlichen Menge an Gas ($\Delta V$Netz) gemäß Gleichung (8) berechnet wird und
b) in Verfahrensschritt 1. b) die Mengen (VA) an den Ausspeisestellen nach dem durch die Gleichung (12) beschriebenen Korrekturverfahren ermittelt werden.

Es handelt sich um ein Korrekturverfahren für die Mengen an den Ausspeisestellen, welches im Folgenden beschrieben wird:

Bei der Anwendung von Gleichung (3) wird sich in der Regel zeigen, dass die Bilanz nicht exakt aufgeht. Grund hierfür sind im Wesentlichen Ungenauigkeiten bei der Bestimmung der Verbrauchsmengen mittels Lastprofilen. Der Einfluss von Messunsicherheiten bei den Einspeisemengen sowie der Einfluss der Netzatmung sind vergleichsweise gering. Zur Verbesserung der Genauigkeit wird daher das folgende Korrekturverfahren angewendet

Durch Umstellen von Gleichung (3) lässt sich die tatsächliche Gesamtmenge bestimmen, die aus dem Netz ausgespeist wird.

$$V_A^\bullet = V_E - \Delta V_{Netz} \qquad (9)$$

Die Differenz zwischen der tatsächlichen und der geschätzten Ausspeisemenge wird in dem folgenden Korrekturterm berücksichtigt

$$\Delta V_{Kor} = V_A^\bullet - V_A \qquad (10)$$

Um nun die Mengen an den einzelnen Ausspeisestellen mit größtmöglicher Genauigkeit zu bestimmen, wird die gesamte zu korrigierende Menge $\Delta V_{kor}$ proportional zu den geschätzten Mengen $V_{A,i}$ auf die Ausspeisestellen verteilt. Dies wird durch folgende Formel ausgedrückt:

$$V_{A,i}^\bullet = \left(1 + \frac{\Delta V_{Kor}}{V_{A,SLP}}\right) \cdot V_{A,i} \qquad (11)$$

Durch Einsetzen der Gleichungen (9) und (10) In Gleichung (11) ergibt sich die korrigierte Menge an den Ausspeisestellen zu:

$$V_{A,i}^{\bullet} = \left(1 + \frac{V_E - V_A - \Delta V_{Netz}}{V_{A,}}\right) \cdot V_{A,i} \qquad (12)$$

Eine vorteilhafte Weiterbildung ist dadurch gekennzeichnet, dass

a) an allen Netzknoten die Mengenanteile der eingespeisten Brenngase auf Basis einer Mengenbilanz sowie die zugehörigen Laufzeiten ermittelt werden und
b) eine Zuordnung des an der Ausspeisestelle berechneten Brennwertes ($H_{S,A}$) zu den Brennwerten ($H_{S,E}$) an den Einspeisestellen vorgenommen wird.

Dies ist vor allem in Bezug auf die Transparenz und die Überprüfungsmöglichkeit des beschriebenen Verfahrens wichtig. Werden beispielsweise die in Rechnung gestellten Brennwerte von einem Kunden in Frage gestellt, so lässt sich anhand des oben beschriebenen Verfahrens nachvollziehen, wie sich der Brennwert an der Ausspeisestelle aus den einzelnen Brennwerten der Brenngase an den Einspeisestellen zusammensetzt. Dabei kann unterstellt werden, dass sich der Brennwert einer Mischung aus den Brennwerten der Brenngase an den Einspeisestellen proportional zum Mengenverhältnis ergibt. Die Korrektheit der Brennwerte der Brenngase an den Einspeisestellen wird durch Messung mit geeichten Brennwertmessgeräten - in der Regel Prozessgaschromatografen - sichergestellt und kann jederzeit von den zuständigen Behörden überprüft werden.

Erst durch das mittels der Gleichungen (7) bis (12) beschrieben Korrekturverfahren lassen sich die Mengen an den Ausspeisestellen mit der Güte bestimmen, die erforderlich ist um eine genaue Brennwertverfolgung - wie nachfolgend beschrieben - zu realisieren.

Für die Bestimmung der zeitlichen und örtlichen Verteilung des Brennwertes im Netz wird eine sogenannte Brennwertzuordnung eingesetzt Für die Brennwertzuordnung wird der zum Zeitpunkt T an einem Netzknoten $K_i$ (Ausspeiseknoten zählen hierbei ebenfalls als Netzknoten und werden somit gleichbehandelt) vorherrschende Brennwert zu einem bestimmten Zeitpunkt $t$ $\dot{H}_{S,Ki}(t)$ durch eine gewichtete Summe der Brennwerte $\overline{H}_{S,Ki}(\hat{t})$ des an den Einspeisestellen $E_i$ zu ggf. abweichenden Zeitpunkten $\hat{\tau} < t$ eingespeisten Gases dargestellt, d.h.

$$\hat{H}_{S,Ki}(t) = \sum_{i=1}^{N_E} \sum_{\hat{t}=1}^{t} x_i(\hat{t}) \cdot \overline{H}_{S,Ei}(\hat{t}), 0 \leq x_i(\hat{t}) \leq 1. \qquad \text{N1}$$

Dabei wird davon ausgegangen, dass an den Einspeisestellen $E_i$ die über einen Zeitraum $\Delta\tau$ (z. B.$\Delta\tau$=1h) gemittelten Brennwerte $\overline{H}_{S,Ei}(\hat{t})$ zu den diskreten Zeitpunkten $\hat{\tau}$ =1,2,...vorliegen - diese Archivierung ist bei der Protokollierung der Brennwerte in Prozessgaschromatographen (PGC) üblich.

Gesucht wird also der Brennwert des zum Zeitpunkt $t$ an dem Netzknoten K anliegenden Brennwerts $\hat{H}_{S,Ki}(t)$ unter Verwendung von Gleichung N1.

Die zur Bestimmung des Brennwerts benötigten Gewichte (entsprechend den jeweiligen Anteile der eingespeisten Gase) $x_i(\hat{\tau})$ werden dabei mit Hilfe eines neuartigen Verfahrens ermittelt: Dazu wird ein zum Zeitpunkt $t$ an dem Netzknoten K (aus Gründen der besseren Lesbarkeit wird im Folgenden auf den Index i verzichtet) aus dem Gasnetz geströmtes Gaspaket $p_K^0(t)$ zeitlich rückwärts durch das Gasnetz verfolgt, bis es das Gasnetz vollständig durch die Einspeisestellen verlassen hat.

Zur Rückverfolgung können die Strömungsgeschwindigkeiten innerhalb der Rohre, die Volumenströmen oder weiterer, die Dynamik des Gases im Netz beschreibende Größen verwendet werden - entweder durch Messung oder durch Berechnung. Die Eingangsgrößen der Simulationsrechnung sind im Folgenden aufgeführt

Eingangsgrößen:

- Brennwerte an den Einspeisestellen (gemessen) $H_{sE,i}$
- Mengen an den Einspeisestellen (gemessen) $V_{E,i}$

- Mengen an allen Ausspeisestellen (korrigiert) $V_{A,i}^{\bullet}$

- Leitungsdrücke $p$
- Netztopologie: Topologische Daten des Netzes, u. a. Leitungslängen, Leitungsdurchmesser, Rohrrauhigkeit

Als Ergebnis der Simulation werden z.B. die Strömungsgeschwindigkeiten und daraus, durch Anwendung des o. g. Verfahrens die Brennwerte an allen Netzknoten als Stundenmittelwerte bestimmt.

Da bei der Anwendung von Gleichung (6) zunächst mit einem mittleren Brennwert gerechnet wurde, können nun die Gleichungen (6) bis (12) unter Verwendung der im Schritt ii) ermittelten Brennwerte neu ausgeführt werden. Damit lässt sich die Genauigkeit der ausgespeisten Mengen und letztendlich die Genauigkeit der Brennwerte der ausgespeisten Mengen verbessern. In der Regel genügt ein zusätzlicher Iterationsschritt um die erforderliche Genauigkeit zu erreichen. Bei Bedarf können weitere Iterationsschritte zur weiteren Erhöhung der Genauigkeit durchgeführt werden.

Bei der Ermittlung der Brennwerte für die jeweilige Ausspeisestelle werden vier Fälle unterschieden:

a) Ausspeisestellen denen eindeutig der Brennwert einer Einspeisestelle zugeordnet werden kann. Dieser Fall ist für die meisten Ausspeisestellen gegeben (i. d. R. zu mehr als 90%)

b) Ausspeisestellen an denen es zu zeitlichen Schwankungen zwischen zwei Einspeisebrennwerte kommen kann (Pendelzonen)

c) Ausspeisestellen bei denen sich der Brennwert aus Mischungen zweier (oder mehrerer) Einspeisebrennwerte zusammensetzt (Mischzonen)

d) Ausspeisestellen, deren nachgeschalteten Verteilnetze untereinander vermascht, d. h. an einer oder mehreren Stellen verbunden sind.

zu a) In diesem Fall können als Brennwerte für die Ausspeisestelle, d. h. für Abrechnungszwecke direkt die Einspeisebrennwerte (Stundenwerte) zugrunde gelegt werden. Ein zeitlicher Versatz (Laufzeitdifferenz) wird entsprechend berücksichtigt.

zu b) Pendelzonen werden über die Simulationsrechnung identifiziert (Umkehr der Strömungsrichtung). Zusätzlich wird unter Berücksichtigung der Unsicherheit der Simulationsrechnung ein Bereich festgelegt, in dem es zu einer Pendelzone kommen kann. Jede Ausspeisestelle, die sich in diesem Bereich befindet, wird mit dem niedrigeren der beiden Einspeisebrennwerte abgerechnet ("best Abrechnung" - im Zweifel zu Gunsten des Kunden).

zu c) Ergibt sich die Abnahmemenge an einer Ausspeisestelle aus der Mischung zweier Gasströme mit unterschiedlichen Einspeisebrennwerten, so kann der Mischungsbrennwert aus dem Mischungsverhältnis berechnet werden.

zu d) Im Falle von vermaschten Netzen wird der niedrigste Brennwert zugrunde gelegt, der an der zugehörigen Ausspeisestelle ermittelt wurde.

Bei der Bestimmung der Brennwerte an den Ausspeisestellen bzw. der Abrechnungswerte wird für jeden einzelnen Stundenwerte die Aufteilung in die Fälle a) bis d) gemacht und entsprechend im Ergebnisbericht gekennzeichnet. Aus den Stundenwerten wird dann der Monatswert als mengengewichteter Mittelwert bestimmt.

Eine weitere vorteilhafte Weiterbildung ist dadurch gekennzeichnet, dass a) ein Bilanzausgleich nach Gleichung (15) durchgeführt wird, so dass die aus- und eingespeisten Energiemengen übereinstimmen.

Damit die Energiebilanz des Netzbetreibers ausgeglichen ist, wird ein Bilanzausgleich der ein- und ausgespeisten Energiemenge vorgenommen. Die Korrektur erfolgt anhand der Brennwerte an den Ausspeisestellen. Dabei darf allerdings die Korrektur der Brennwerte nicht mehr als 2 % betragen.

Die zu korrigierende Energiemenge ergibt sich aus

$$\Delta E_{Kor} = \sum_i V_{E,i} \cdot H_{sE,i} - \sum_i V_{A,i} \cdot H_{sA,i} - \sum_i \Delta V_{Netz,i} \cdot H_{s,m,i} \qquad (13)$$

Die korrigierten Brennwerte ergeben sich zu

$$H_{s,Al}^{\bullet} = \left(1 + \frac{\Delta E_{Kor}}{\sum_i V_{A,l} \cdot H_{sA,l}}\right) \cdot H_{sA,l} \tag{14}$$

bzw. durch Einsetzen von Gleichung (10) in Gleichung (11) ergibt sich

$$H_{s,Al}^{\bullet} = \left(1 + \frac{\sum_i V_{E,l} \cdot H_{sE,l} - \sum_i V_{A,l} \cdot H_{sA,l} - \sum_i \Delta V_{Netz,l} \cdot H_{s,m,l}}{\sum_i V_{A,l} \cdot H_{sA,l}}\right) \cdot H_{sA,l} \tag{15}$$

Eine weitere vorteilhafte Weiterbildung der Erfindung besteht darin

a) im Verfahrensschritt 1.b) die Mengen einzelner Verbraucher zu den verschiedenen Lastprofiltypen mittels Gaszählern mit elektronischer Datenfernübertragung gemessen werden und somit die Koeffizienten A, B, C, D von Gleichung (1) regelmäßig neu bestimmt werden.

Die Erfindung wird im Folgenden anhand eines bevorzugten Ausführungsbeispiels Ausführungsformen des erfindungsgemäßen Verfahrens im Zusammenhang mit der Zeichnung näher erläutert.
Die Zeichnung zeigt in:

Fig. 1 ein Gasnetz nach dem Stand der Technik in einer schematische Darstellung;
Fig. 2 in einem Diagramm den Vergleich der über Standard-Lastprofile ermittelten Ausspeisemengen (korrigiert/unkorrigiert) mit gemessenen Werten, wobei die korrigierten Werte nach Gleichung (8) bestimmt wurden.
Fig. 3 ein schematisches Blockdiagramm zwecks Darstellung des erfindungsgemäßen Verfahrens.

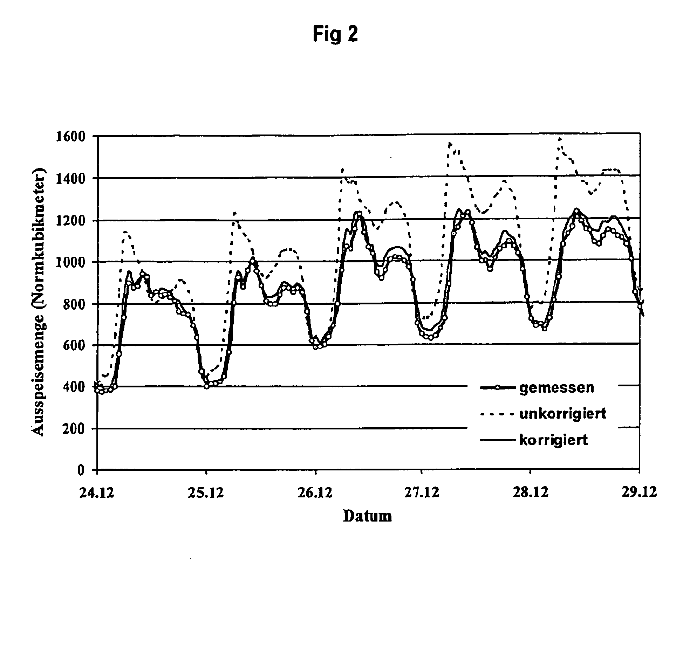

Anhand von Fig. 2 wird das Verfahren nach Anspruch 2 verdeutlicht. Am Beispiel eines Ausspeiseknotens werden hier die mittels Lastprofilen bestimmten Mengen (unkorrigiert / korrigiert) mit Messwerten verglichen. Der Vergleich zeigt, dass das beschriebene Korrekturverfahren zu einer erheblichen Verbesserung führt.
Bei größeren Endverbrauchern, z. B. Industrie- oder Gewerbebetriebe, werden die Mengen teilweise auch mit Gaszähler mit elektronischer Datenfernübertragung gemessen. Steht diese Information zu Verfügung, so kann die Menge $V_A$ aufgeteilt werden in Mengen, die mittels Zähler messen werden und Mengen die über Lastprofile geschätzt werden. Durch Berücksichtigung dieser Information lässt sich die Genauigkeit des beschriebenen Korrekturverfahrens weiter verbessern.

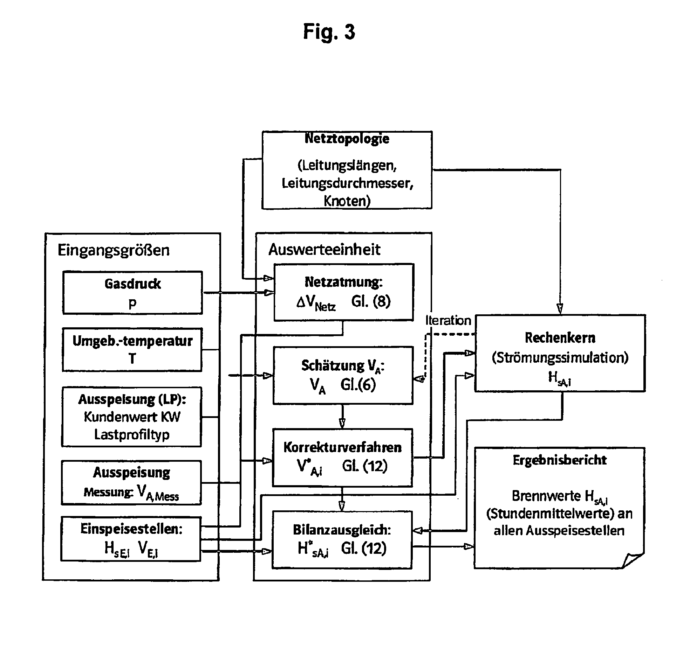

Fig. 3 -zeigt die Datenflüsse des Verfahrens. Die zu berechnenden Größen und die verwendeten Gleichungen werden dargestellt. Die Ein- und Ausgangsgrößen der jeweiligen Verfahrensschritte werden durch Pfeile dargestellt.

**Formelzeichen**

E      Energie in kWh
$H_s$    Brennwert in kWh/m$^3$
p      Druck in bar
T      absolute Temperatur in K
$\vartheta$      relative Temperatur in °C
V      Volumen in m$^3$
$t$      aktuell betrachteter Zeitpunkt, normiert
r      beliebiger Zeitpunkt, normiert
A      Sigmoidkoeffizient in kWh
B      Sigmoidkoeffizient in °C

C    Sigmoidkoeffizient, einheitenlos
D    Sigmoidkoeffizient in kWh

**Indizes**

A    Ausspeisung
E    Einspeisung
K    betrachteter Netzknoten (schließt auch Ausspelseknoten mit ein)
i    Netzknotenindex
n    Normbedingungen ($T_n$ = 273,15 K, $p_n$ = 1,01325 bar)
m    Mittelwert

**Patentansprüche**

**1.**  Verfahren zur Bestimmung des Brennwertes von Brenngas, insbesondere von Erdgas, in Gasnetzen, insbesondere in Regional- oder Verteilnetzen mit mindestens zwei Einspeisestellen, einer Mehrzahl von Netzknoten und mehreren Ausspeisestellen,
**dadurch gekennzeichnet, dass**

a) die Brennwerte ($H_{S,E}$) und Mengen ($V_E$) an den Einspeisestellen des Gasnetzes gemessen werden,
b) die Mengen ($V_A$) an den Ausspeisestellen des Gasnetzes auf Basis von Lastprofilen aus der Energiemenge $Q_h$ und dem Brennwert $H_{s,A}$ gemäß der Gleichung

$$V_{A,i} = \frac{Q_{h,i}}{H_{sA,i}} \tag{6}$$

geschätzt und gemäß der Gleichung

$$V_A = \sum_i V_{A,i} \tag{5}$$

summiert werden, wobei sich die Energiemenge $Q_h$ aus der Gleichung

$$Q_h = KW \cdot h(\vartheta) \cdot F(d) \cdot SF(h,\vartheta) \tag{2}$$

ergibt und wobei KW der Kundenwert (in kWh), h(9) der relative Verbrauch gemäß der Sigmoid-Funktion, F(d) der Wochentagsfaktor und SF(h, 9) der Stundenfaktor ist,
c) die in den Verfahrensschritten a) und b) ermittelten Werte zusammen mit topologischen Daten des Gasnetzes einer Auswerteeinheit zugeführt und die Brennwerte ($H_{S,A}$) an mindestens einer Ausspeisestelle rechnerisch bestimmt werden.

**2.**  Verfahren nach Anspruch 1,
**dadurch gekennzeichnet, dass**

a) an allen Netzknoten die Mengenanteile der eingespeisten Brenngase auf Basis einer Mengenbilanz sowie die zugehörigen Laufzeiten ermittelt werden und
b) eine Zuordnung des an der Ausspeisestelle berechneten Brennwertes ($H_{S,A}$) zu den Brennwerten ($H_{S,E}$) an den Einspeisestellen vorgenommen wird.

**3.**  Verfahren nach Anspruch 2,

**dadurch gekennzeichnet, dass**
in Verfahrensschritt 2 b) der zum Zeitpunkt t an den Netzknoten anliegende Brennwert auf Basis der Anteile $\hat{x_i}(\tau)$ der zu den Zeitpunkten $\tau < t$ an den Einspeisestellen anliegenden Brennwerten $\overline{H}_{S,Ei}(\hat{\tau})$ ermittelt wird, wobei ein aus dem Gasnetz geströmtes Gaspaket zeitlich rückwärts durch das Gasnetz verfolgt wird bis es das Gasnetz vollständig durch die Einspeisestellen $E_i$ verlassen hat.

4. Verfahren nach einem der Ansprüche 1 bis 3,
   **dadurch gekennzeichnet, dass**

   a) der mittlere Netzdruck ($p_m$) durch Messung des Druckes an repräsentativen Stellen in dem Gasnetz ermittelt wird und die zeitliche Änderung der im Gasnetz befindlichen Menge an Gas ($\Delta V_{Netz}$) gemäß Gleichung (8) berechnet wird und
   b) in Verfahrensschritt 1. b) die Mengen ($V_A$) an den Ausspeisestellen nach dem durch die Gleichung (12) beschriebenen Korrekturverfahren ermittelt werden.

5. Verfahren nach Anspruch 4,
   **dadurch gekennzeichnet, dass**

   a) ein Bilanzausgleich nach Gleichung (15) durchgeführt wird, so dass die aus- und eingespeisten Energiemengen übereinstimmen.

6. Verfahren nach einem der Ansprüche 1 bis 5,
   **dadurch gekennzeichnet, dass**

   a) im Verfahrensschritt 1. b) die Mengen einzelner Verbraucher zu den verschiedenen Lastprofiltypen mittels Gaszählern mit elektronischer Datenfernübertragung gemessen werden und somit die Koeffizienten A, B, C, D von Gleichung (1) regelmäßig neu bestimmt werden.

## Claims

1. Method for determining the superior calorific value of fuel gas, especially of natural gas, in gas networks, especially in regional or distribution networks with at least two feed-in points, a plurality of network nodes and several feed-out points,
   **characterised in that**

   a) the superior calorific values ($H_{S,E}$) and volumes ($V_E$) are measured at the feed-in points of the gas network,
   b) the volumes ($V_A$) at the feed-out points of the gas network are estimated on the basis of load profiles from the energy quantity $Q_h$ and the superior calorific value $H_{S,A}$ in accordance with the equation

$$V_{A,i} = \frac{Q_{h,i}}{H_{sA,i}} \qquad (6)$$

and added up in accordance with the equation

$$V_A = \sum_i V_{A,i} \qquad (5)$$

with the energy quantity $Q_h$ resulting from the equation

$$Q_h = KW \cdot h(\vartheta) \cdot F(d) \cdot SF(h, \vartheta) \qquad (2)$$

and KW being the customer value (in kWh), h($\vartheta$) the relative consumption in accordance with the sigmoid function, F(d) the week-day factor and SF(h, $\vartheta$) the hourly factor,

c) the values determined in method steps a) and b), together with the topological data of the gas network, are supplied to an evaluation unit and the superior calorific values ($H_{S,A}$) are determined arithmetically at a minimum of one feed-out point.

**2.** Method according to claim 1,
**characterised in that**

a) the volume portions of the fuel gases fed in are determined at all network nodes on the basis of a volume balance and the associated run times, and
b) an assignment is made of the superior calorific value ($H_{S,A}$) calculated at the feed-out point to the superior calorific values ($H_{S,E}$) at the feed-in points.

**3.** Method according to claim 2,
**characterised in that**
in method step 2b) the superior calorific value present at the network nodes is determined at the time t on the basis of the portions $x_i(\hat{\tau})$ of the superior calorific values $\overline{H}_{S,E_i}(\hat{\tau})$ present at the feed-in points at the times $\hat{\tau} < t$, with a gas package that has flowed out of the gas network being tracked backwards in time through the gas network until it has fully left the gas network through the feed-in points $E_i$.

**4.** Method according to one of claims 1 to 3,
**characterised in that**

a) the medium network pressure ($p_m$) is determined by measuring the pressure at representative points in the gas network and the change over time of the quantity of gas ($\Delta V_{network}$) present in the network is calculated in accordance with equation (8) and
b) in method step 1 b) the volumes ($V_A$) at the feed-out points are determined by way of the correction method described by equation (12).

**5.** Method according to claim 4,
**characterised in that**

a) a balancing adjustment according to equation (15) is performed so that the energy quantities fed out and fed in are the same.

**6.** Method according to one of claims 1 to 5,
**characterised in that**

a) in method step 1 b) the volumes of individual consumers load profile types are measured by means of gas meters with remote electronic data transmission and thus the coefficients A, B, C, D of equation (1) are regularly determined new.

**Revendications**

**1.** Procédé pour déterminer le pouvoir calorifique supérieur d'un gaz combustible, en particulier de gaz naturel, dans des réseaux de gaz, en particulier des réseaux régionaux ou des réseaux de distribution avec au moins deux points d'injection, une multitude de noeuds et plusieurs points de livraison,
**caractérisé par le fait que**

a) les pouvoirs calorifiques supérieurs ($H_{S,E}$) et quantités ($V_E$) sont mesurés aux points d'injection dans le réseau de gaz,
b) les quantités ($V_A$) aux points de livraison du réseau de gaz sont estimées, sur la base de profils de demande, dérivés de la quantité d'énergie ($Q_h$) et du pouvoir calorifique supérieur ($H_{S,A}$), suivant l'équation

$$V_{A,i} = \frac{Q_{h,i}}{H_{sA,i}} \tag{6}$$

et totalisées suivant l'équation

$$V_A = \sum_i V_{A,i} \tag{5}$$

la quantité d'énergie $Q_h$ découlant de l'équation

$$Q_h = KW \cdot h(\vartheta) \cdot F(d) \cdot SF(h,\vartheta) \tag{2}$$

où *KW* étant la valeur attribuée au client (en kWh), $h(\vartheta)$ la consommation relative suivant la fonction sigmoïde, *F(d)* le facteur journalier et SF(h, $\vartheta$) le facteur horaire,

c) les valeurs déterminées lors des phases a) et b) du procédé sont amenées, ensemble avec des données topologiques du réseau de gaz, à une unité d'exploitation de données, et les pouvoirs calorifiques supérieurs $(H_{S,A})$ sont déterminé par calcul à au moins un point de livraison.

2. Procédé suivant la revendication 1,
   **caractérisé par le fait que**

   a) au niveau de tous les noeuds, les parts des volumes des gaz combustibles injectés sont déterminées sur la base d'un bilan des volumes, et sont également déterminées les durées de parcours correspondantes, et
   b) une attribution du pouvoir calorifique supérieur $(H_{S,A})$ calculé au point de livraison aux pouvoirs calorifiques supérieurs $(H_{S,E})$ aux points d'injection est effectuée.

3. Procédé suivant la revendication 2,
   **caractérisé par le fait que**
   lors de phase 2 b) du procédé, le pouvoir calorifique supérieur présent aux noeuds au moment t est déterminé sur la base des parts $x_i(\hat{\tau})$ des pouvoirs calorifiques supérieurs $\overline{H}_{S,INi}(\hat{\tau})$, présents aux points d'injection au moment $i <$ *t,* où un paquet de gaz issu du réseau de gaz est tracé temporellement en arrière à travers le réseau de gaz jusqu'à ce qu'il ait complètement quitté le réseau de gaz par les points d'injection $E_i$.

4. Procédé suivant l'une des revendications 1 à 3,
   **caractérisé par le fait que**

   a) la pression moyenne dans le réseau (p $_m$) est déterminée par mesurage de la pression aux points représentatifs dans le réseau de gaz, et le changement dans le temps du volume de gaz présent dans le réseau de gaz $(\Delta V_{network})$ est calculé suivant l'équation (8), et
   b) lors de la phase 1 b), les quantités $(V_A)$ aux points de livraison sont déterminées suivant la procédure de correction, décrite par l'équation (12).

5. Procédé suivant la revendication 4,
   **caractérisé par le fait que**

   a) il est procédé à un équilibrage du bilan suivant l'équation (15) pour assurer que les quantités livrées cadrent avec les quantités injectées.

6. Procédé suivant l'une des revendications 1 à 5,
   **caractérisé par le fait que**

a) lors de la phase 1 b) du procédé, les quantités de clients individuels des différents types de profil de demande sont mesurées à l'aide de compteurs à gaz, dotés d'un système d'échange de données informatisées, et que les coefficients A, B, C et D de l'équation (1) sont de ce fait régulièrement recalculés.

# Fig. 1

# Fig 2

# Fig. 3

Netztopologie

(Leitungslängen,
Leitungsdurchmesser,
Knoten)

Eingangsgrößen

Gasdruck
p

Umgeb.-temperatur
T

Ausspeisung (LP):
Kundenwert KW
Lastprofiltyp

Ausspeisung
Messung: $V_{A,Mess}$

Einspeisestellen:
$H_{sE,i}$  $V_{E,i}$

Auswerteeinheit

Netzatmung:
$\Delta V_{Netz}$   Gl. (8)

Iteration

Schätzung $V_A$:
$V_A$   Gl.(6)

Korrekturverfahren
$V^*_{A,i}$   Gl. (12)

Bilanzausgleich:
$H^*_{sA,i}$   Gl. (12)

Rechenkern
(Strömungssimulation)
$H_{sA,i}$

Ergebnisbericht

Brennwerte $H_{sA,i}$
(Stundenmittelwerte) an
allen Ausspeisestellen

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **ECKART et al.** *Rechnergestützte Zuordnung von an den Einspeisepunkten eines Ferngasnetzes vorgenommenen Brennwertmessungen zu den an Übergabestationen entnommenen Gasmengen,* 1983, vol. 124, 157-164

- **ALTFELD, K. ; BÖDEKER, J. ; FRIELING, H. ; SCHLEY, P. ; UHRIG, M.** Modelling of Gas Flow in Pipelines for Tracking Gas Quality. *Proceedings of the International Gas Research Conference, Paris,* 2008
- **HELLWEG, M.** *Entwicklung und Anwendung parametrisierter Standard-Lastprofile,* 2003